# EUROPEAN PATENT SPECIFICATION

(11) **EP 2 882 402 B1**
(45) Date of publication and mention of the grant of the patent: **10.10.2018**
(21) Application number: 13747663.6
(22) Date of filing: 07.08.2013
(51) Int. Cl.: A61K 8/03, A61Q 5/06, A61Q 5/12, A61K 8/31, A61K 8/34, A61K 8/37, A61K 8/81, A61K 8/04

(54) **TWO-PHASE COSMETIC COMPOSITION AND PUMP BOTTLE CONTAINING IT**
ZWEIPHASIGE KOSMETISCHE ZUSAMMENSETZUNG UND PUMPFLASCHE DAMIT
COMPOSITION COSMÉTIQUE À DEUX PHASES ET FLACON À POMPE LA CONTENANT

(30) Priority: 07.08.2012 FR 1257669; 16.11.2012 US 201261727154 P
(43) Date of publication of application: 17.06.2015
(73) Proprietor: L'Oréal, 75008 Paris (FR)
(72) Inventor: STEFANI, Raquel, F-92600 Asnieres (FR); GAWTREY, Jonathan, F-92100 Boulogne (FR)
(74) Representative: Casalonga
(86) International application number: PCT/EP2013/066578
(87) International publication number: WO 2014/023782

(56) References cited:
- EP-A1- 0 418 676
- EP-A1- 2 022 466
- EP-A1- 2 022 467
- WO-A1-02/060397
- DE-A1- 19 703 475
- US-A1- 2012 064 137

## Description

The present invention relates to a cosmetic composition, preferably conditioned in a pump bottle, comprising at least two mutually immiscible liquid phases, and to its use for shaping and/or holding the hairstyle.

In the field of shaping and/or holding the hairstyle, hair products to be sprayed, such as sprays, are generally used.

They are essentially formed from an alcoholic and/or aqueous solution and from one or more materials, generally polymer resins (known as fixing agents), whose function is to form welds between the hairs, as a mixture with various cosmetic adjuvants. These hair products may or may not be pressurized.

Moreover, hair products also exist for giving the hair sheen. These products are often compositions to be sprayed, and are not intended for fixing the hair.

Products in a pump bottle intended to provide both sheen and fixing to the hair exist on the market. These are one-phase products. However, they give a poor level of fixing.

There is thus a need to develop novel products that afford greater levels of fixing, while at the same time maintaining good sheen.

The Applicant has found, surprisingly and advantageously, that the use of two preparations, one comprising at least one ionic or nonionic fixing polymer that is soluble in an aqueous-alcoholic or alcoholic medium, and a particular amount of water, and the other being a fatty phase that is insoluble in the said aqueous-alcoholic or alcoholic medium, makes it possible to obtain a product comprising at least two mutually immiscible liquid phases, known as a two-phase product, which, when sprayed for example from a pump bottle, gives fixing, styling and sheen.

One subject of the invention is thus a cosmetic composition comprising two or more than two separate liquid phases:
(i) one of the liquid phases comprising an alcoholic or aqueous-alcoholic medium and one or more fixing polymers that are soluble in the said medium, and
(ii) the other of the liquid phases being a fatty phase that is insoluble in phase (i) and comprising one or more fatty substances.
the phase (i) comprising from 0 to 50% by weight of water relative to the total weight of phase (i).

This composition differs from emulsions in that, when at rest and at room temperature, the two phases are visually distinct instead of being emulsified one in the other. Thus, the two phases are separated at rest by a single interface, whereas, in emulsions, one of the phases is dispersed in the other in the form of a multitude of droplets, and the interfaces are therefore multiple, these interfaces generally being stabilized with emulsifying surfactants and/or emulsifying polymers. The use of two-phase compositions necessitates prior shaking in order to form an extemporaneous emulsion. This emulsion must be of sufficient quality and stability to enable a homogeneous application of the two phases, but such that, when at rest, the two phases become rapidly separated and regain their initial state, this phenomenon being more commonly known as "dephasing".

This particular combination makes it possible to obtain a two-phase cosmetic composition which preferably has an acceptable dephasing time after shaking, for example ranging from 5 minutes to 60 minutes, and which affords sheen and fixing with a rapid drying time.

The present invention also relates to a pump bottle containing the cosmetic composition according to the invention.

A subject of the invention is also a process for shaping and/or holding the hairstyle, comprising the spraying onto the hair of the cosmetic composition according to the invention.

Another subject of the invention is the use of the cosmetic composition sprayed from a pump bottle, for shaping and/or holding the hairstyle.

Other subjects, characteristics, aspects and advantages of the invention will become even more clearly apparent on reading the description and examples that follow.

In that which follows and unless otherwise indicated, the limits of a range of values are included within this range, in particular in the expressions "between" and "ranging from ... to ...".

Moreover, the expression "at least one" used in the present description is equivalent to the expression "one or more".

According to the invention, the cosmetic composition comprises two or more than two, preferably two, separate liquid phases:
(i) one of the liquid phases comprising an alcoholic or aqueous-alcoholic medium and one or more fixing polymers that are soluble in the said medium, and
(ii) the other of the liquid phases being a fatty phase that is insoluble in phase (i), i.e. in the said medium, and comprising one or more fatty substances.
the phase (i) comprising from 0 to 50% by weight of water relative to the total weight of phase (i).

The cosmetic composition according to the invention comprises at least one alcoholic or aqueous-alcoholic liquid phase (i) and a distinct liquid fatty phase (ii). These two phases are distinct, i.e. they are visible one above the other at rest at room temperature (25°C). Preferably, the fatty phase (ii) is above the alcoholic or aqueous-alcoholic phase (i).

The two phases have a different density. The alcoholic or aqueous-alcoholic phase has a density at 20°C preferably ranging from 0.800 to 0.950 and better still from 0.850 to 0.900. The fatty phase has a density at 20°C preferably ranging from 0.700 to 0.900 and better still from 0.750 to 0.880.

The density at 20°C is measured with a standard densimeter, for example a Mettler-Toledo DE 45 machine.

Preferably, the weight ratio of phase (i) to phase (ii) ranges from 0.1 to 20 and better still from 0.8 to 20.

According to the invention, phase (i) comprises an alcoholic or aqueous-alcoholic medium and one or more fixing polymers that are soluble in the said medium.

Preferably, the alcoholic or aqueous-alcoholic medium comprises one or more C₁-C₆ alcohols.

Among these alcohols, mention may be made of ethanol, isopropanol, poly alcohols such as diethylene glycol, glycol ethers, and glycol mono alkyl ethers, diethylene glycol mono alkyl ethers, propylene glycol mono alkyl ethers or dipropylene glycol mono alkyl ethers. Ethanol is particularly preferred.

The C₁-C₆ alcohol(s) are present in an amount preferably ranging from 10% to 95% by weight and better still from 40% to 90% by weight relative to the total weight of phase (i).

Phase (i) comprises from 0 to 50% by weight and better still from 10% to 50% by weight of water relative to the total weight of phase (i).

As indicated previously, the cosmetic composition according to the invention comprises one or more fixing polymers that are soluble in the alcoholic or aqueous-alcoholic medium.

The term "soluble in the alcoholic or aqueous-alcoholic medium" means a weight solubility in the alcoholic or aqueous-alcoholic medium of greater than 1% and even more preferentially greater than 5% at room temperature (25°C) and at atmospheric pressure (760 mmHg, i.e. 1.013 × 10⁵ Pa).

The term "fixing polymer" means any polymer capable of conferring a shape on a head of hair or of maintaining a head of hair in a given shape.

The fixing polymer(s) used in the hair lacquer according to the invention are selected from anionic, cationic, amphoteric and nonionic fixing polymers, and mixtures thereof.

Anionic polymers that may be mentioned include polymers comprising groups derived from carboxylic acids, sulfonic acids or phosphoric acids, and having a number-average molecular mass of between 500 and 5 000 000.

The carboxylic groups are provided by unsaturated monocarboxylic or dicarboxylic acid monomers such as those corresponding to the formula: 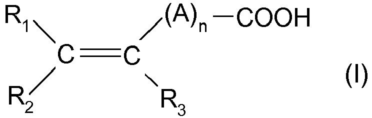 in which n is an integer from 0 to 10, A denotes a methylene group, optionally linked to the carbon atom of the unsaturated group or to the neighbouring methylene group when n is greater than 1 via a heteroatom such as oxygen or sulfur, R₁ denotes a hydrogen atom or a phenyl or benzyl group, R₂ denotes a hydrogen atom, an alkyl group comprising from 1 to 4 carbon atoms or a carboxyl group, and R₃ denotes a hydrogen atom, an alkyl group comprising from 1 to 4 carbon atoms or a -CH₂-COOH, phenyl or benzyl group.

In formula (I) above, the alkyl group comprising from 1 to 4 carbon atoms may in particular denote methyl and ethyl groups.

The anionic fixing polymers containing carboxylic groups that are preferred are:
A) acrylic or methacrylic acid homo- or copolymers, or salts thereof and in particular the products sold under the names Versicol® E or K by the company Allied Colloid and Ultrahold® by the company BASF, the copolymers of acrylic acid and of acrylamide sold in the form of their sodium salts under the names Reten 421, 423 or 425 by the company Hercules, the sodium salts of polyhydroxycarboxylic acids;
B) copolymers of acrylic or methacrylic acid with a monoethylenic monomer such as ethylene, styrene, vinyl esters, acrylic or methacrylic acid esters, optionally grafted onto a polyalkylene glycol such as polyethylene glycol and optionally crosslinked. Such polymers are described in particular in French patent 1 222 944 and German patent application 2 330 956, the copolymers of this type comprising an optionally N-alkylated and/or hydroxyalkylated acrylamide unit in their chain, as described in particular in Luxembourgian patent applications 75370 and 75371 or provided under the name Quadramer by American Cyanamid. Mention may also be made of copolymers of acrylic acid and of C₁-C₄ alkyl methacrylate and terpolymers of vinylpyrrolidone, of acrylic acid and of methacrylate of C₁-C₂₀ alkyl, for example lauryl methacrylate, such as the product sold by the company ISP under the name Acrylidone® LM and methacrylic acid/ethyl acrylate/tert-butyl acrylate terpolymers such as the product sold under the name Luvimer® 100 P by the company BASF.
   Mention may also be made of methacrylic acid/acrylic acid/ethyl acrylate/methyl methacrylate copolymers as an aqueous dispersion, sold under the name Amerhold® DR 25 by the company Amerchol;
C) crotonic acid copolymers, such as those comprising vinyl acetate or propionate units in their chain and optionally other monomers such as allyl esters or methallyl esters, vinyl ether or vinyl ester of a linear or branched, saturated carboxylic acid with a long hydrocarbon-based chain, such as those containing at least 5 carbon atoms, it being possible for these polymers optionally to be grafted or crosslinked, or alternatively another vinyl, allyl or methallyl ester monomer of an α- or β-cyclic carboxylic acid. Such polymers are described, *inter alia,* in French patent Nos. 1 222 944, 1 580 545, 2 265 782, 2 265 781, 1 564 110 and 2 439 798. Commercial products that come under this category are the resins 28-29-30, 26-13-14 and 28-13-10 sold by National Starch;
D) copolymers of C₄-C₈ monounsaturated carboxylic acids or anhydrides selected from:
   - copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and its esters, the anhydride functions of these copolymers optionally being monoesterified or monoamidated. Such polymers are described, in particular, in US patent Nos. 2 047 398, 2 723 248 and 2 102 113, and GB patent No. 839 805. Commercial products are especially those sold under the names Gantrez® AN or ES by the company ISP;
   - copolymers comprising (i) one or more maleic, citraconic or itaconic anhydride units and (ii) one or more monomers chosen from allyl or methallyl esters optionally comprising one or more acrylamide, methacrylamide, α-olefin, acrylic ester, methacrylic ester, acrylic acid, methacrylic acid or vinylpyrrolidone groups in their chain,
   the anhydride functions of these copolymers optionally being monoesterified or monoamidated.
   These polymers are described, for example, in French patent Nos. 2 350 384 and 2 357 241 by the Applicant;
E) polyacrylamides comprising carboxylate groups.

The homopolymers and copolymers comprising sulfonic groups are polymers comprising vinylsulfonic, styrenesulfonic, naphthalenesulfonic or acrylamidoalkylsulfonic units.

These polymers may especially be selected from:
- polyvinylsulfonic acid salts having a molecular weight of approximately between 1000 and 100 000, and also the copolymers with an unsaturated comonomer such as acrylic or methacrylic acids and esters thereof, and also acrylamide or derivatives thereof, vinyl ethers and vinylpyrrolidone;
- polystyrenesulfonic acid salts such as the sodium salts that are sold for example under the names Flexan® 500 and Flexan® 130 by National Starch. These compounds are described in patent FR 2 198 719;
- polyacrylamidesulfonic acid salts, such as those mentioned in patent US 4 128 631 and more particularly polyacrylamidoethylpropanesulfonic acid sold under the name Cosmedia Polymer HSP 1180 by Henkel.

As another anionic fixing polymer that may be used according to the invention, mention may be made of the branched block anionic polymer sold under the name Fixate G-100 L by the company Lubrizol.

According to the invention, the anionic fixing polymers are preferably selected from copolymers of acrylic acid, such as the acrylic acid/ethyl acrylate/N-tert-butylacrylamide terpolymers sold especially under the name Ultrahold® Strong by the company BASF, copolymers derived from crotonic acid, such as vinyl acetate/vinyl tert-butylbenzoate/crotonic acid terpolymers and the crotonic acid/vinyl acetate/vinyl neododecanoate terpolymers sold especially as Resin 28-29-30 by the company National Starch, polymers derived from maleic, fumaric or itaconic acids or anhydrides with vinyl esters, vinyl ethers, vinyl halides, phenylvinyl derivatives and acrylic acid and esters thereof, such as the methyl vinyl ether/monoesterified maleic anhydride copolymers sold, for example, as Gantrez® by the company ISP, the copolymers of methacrylic acid and methyl methacrylate sold as Eudragit® L by the company Rohm Pharma, the copolymers of methacrylic acid and ethyl acrylate sold as Luvimer® MAEX or MAE by the company BASF, the vinyl acetate/crotonic acid copolymers sold as Luviset CA 66 by the company BASF, the vinyl acetate/crotonic acid copolymers grafted with polyethylene glycol sold as Aristoflex® A by the company BASF, the vinylpyrrolidone/acrylic acid/lauryl methacrylate terpolymers sold under the name Acrylidone® LM by the company ISP, and the polymer sold under the name Fixate G-100 by the company Lubrizol.

Among the anionic fixing polymers mentioned above, it is more particularly preferred in the context of the present invention to use the acrylic acid/ethyl acrylate/N-tert-butylacrylamide terpolymers sold under the name Ultrahold® Strong by the company BASF, the methyl vinyl ether/monoesterified maleic anhydride copolymers sold under the name Gantrez® ES 425 by the company ISP, the copolymers of methacrylic acid and of methyl methacrylate sold under the name Eudragit® L by the company Rohm Pharma, the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, the copolymers of methacrylic acid and of ethyl acrylate sold under the name Luvimer® MAEX or MAE by the company BASF, the vinylpyrrolidone/acrylic acid/lauryl methacrylate terpolymers sold under the name Acrylidone® LM by the company ISP and the polymer sold under the name Fixate G-100 by the company Lubrizol.

The cationic fixing polymers that may be used according to the present invention are preferably chosen from polymers comprising primary, secondary, tertiary and/or quaternary amine groups forming part of the polymer chain or directly attached thereto, and having a molecular weight of between 500 and approximately 5 000 000 and preferably between 1000 and 3 000 000.

Among these polymers, mention may be made more particularly of the following cationic polymers:
(1) homopolymers or copolymers derived from acrylic or methacrylic esters or amides and comprising at least one of the units of the following formulae: 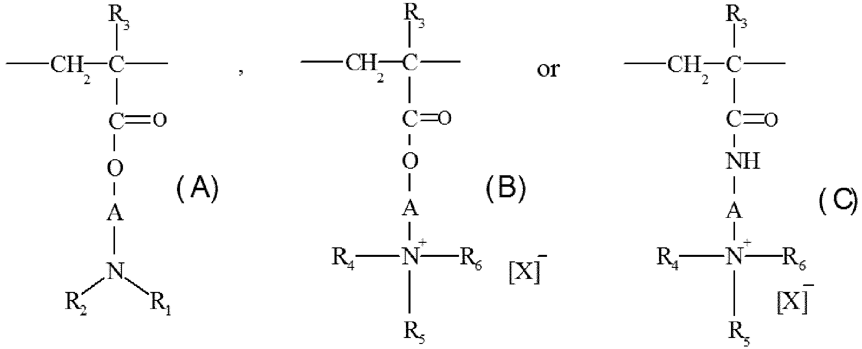 in which:
   R₃ denotes a hydrogen atom or a CH₃ group;
   A is a linear or branched alkyl group comprising from 1 to 6 carbon atoms or a hydroxyalkyl group comprising from 1 to 4 carbon atoms; R₄, R₅ and R₆, which may be identical or different, represent an alkyl group containing from 1 to 18 carbon atoms or a benzyl group;
   R₁ and R₂, which may be identical or different, each represent a hydrogen atom or an alkyl group containing from 1 to 6 carbon atoms; X denotes a methosulfate anion or a halide such as chloride or bromide.
      The copolymers of family (1) also contain one or more units deriving from comonomers that may be chosen from the family of acrylamides, methacrylamides, diacetone acrylamides, acrylamides and methacrylamides which are substituted on the nitrogen with C₁-C₄ alkyl groups, groups derived from acrylic or methacrylic acids or esters thereof, vinyllactams such as vinylpyrrolidone or vinylcaprolactam, and vinyl esters.
      Thus, among these copolymers of family (1), mention may be made of:
      - copolymers of acrylamide and of dimethylaminoethyl methacrylate quaternized with dimethyl sulfate or with a dimethyl halide, such as the product sold under the name Hercofloc® by the company Hercules,
      - copolymers of acrylamide and of methacryloyloxyethyltrimethylammonium chloride, described, for example, in patent application EP-A-080 976 and sold under the name Bina Quat P 100 by the company Ciba Geigy,
      - copolymers of acrylamide and of methacryloyloxyethyltrimethylammonium methosulfate, such as the product sold under the name Reten by the company Hercules,
      - quaternized or non-quaternized vinylpyrrolidone/ dialkylaminoalkyl acrylate or methacrylate copolymers, such as the products sold under the name Gafquat® by the company ISP, such as, for example, Gafquat® 734 or Gafquat® 755, or alternatively the products known as Copolymer® 845, 958 and 937. These polymers are described in detail in French patent Nos. 2 077 143 and 2 393 573,
      - fatty-chain polymers containing a vinylpyrrolidone unit, such as the products sold under the name Styleze W20 and Styleze W10 by the company ISP,
      - dimethylaminoethyl methacrylate/ vinylcaprolactam/ vinylpyrrolidone terpolymers, such as the product sold under the name Gaffix VC 713 by the company ISP, and
      - quaternized vinylpyrrolidone/ dimethylaminopropyl-methacrylamide copolymers, such as the products sold under the name Gafquat® HS 100 by the company ISP;
(2) cationic guar gums, preferably containing quaternary ammonium, such as those described in US patents 3 589 578 and 4 031 307, such as guar gums containing trialkylammonium cationic groups. Such products are sold in particular under the trade names Jaguar C13 S, Jaguar C 15 and Jaguar C 17 by Meyhall;
(3) quaternary copolymers of vinylpyrrolidone and of vinylimidazole, for instance the vinylimidazolium methosulfate/vinylcaprolactam/vinylpyrrolidone terpolymer sold under the name Luviquat Hold by the company BASF;
(4) chitosans or salts thereof; the salts that can be used are, in particular, chitosan acetate, lactate, glutamate, gluconate or pyrrolidonecarboxylate.
   These compounds include the chitosan having a degree of deacetylation of 90.5% by weight which is sold under the name Kytan Brut Standard by the company Aber Technologies, and the chitosan pyrrolidone carboxylate which is sold under the name Kytamer® PC by the company Amerchol;
(5) cationic cellulose derivatives such as copolymers of cellulose or of cellulose derivatives grafted with a water-soluble monomer comprising a quaternary ammonium, and described in particular in patent US 4 131 576, such as hydroxyalkyl celluloses, for instance hydroxymethyl, hydroxyethyl or hydroxypropyl celluloses grafted in particular with a methacryloyloxyethyltrimethylammonium, methacrylamidopropyltrimethylammonium or dimethyl-diallylammonium salt.
   The products sold corresponding to this definition are, more particularly, the products sold under the name Celquat L 200 and Celquat H 100 by the company National Starch;
6) polyvinylamine homopolymers and poly(vinylamine/N-vinylformamide) copolymers.

Among the cationic fixing polymers described above, it will be preferred to use those of family (3), for instance quaternary copolymers of vinylpyrrolidone and of vinylimidazole, and more particularly the vinylimidazolium methosulfate/ vinylcaprolactam/ vinylpyrrolidone terpolymer sold under the name Luviquat Hold by the company BASF, or poly(vinylamine/N-vinylformamide) copolymers such as the product sold under the name Luviquat 9030 by the company BASF.

The amphoteric fixing polymers that can be used in accordance with the invention can be chosen from polymers comprising units B and C distributed randomly in the polymer chain, in which B denotes a unit derived from a monomer comprising at least one basic nitrogen atom and C denotes a unit derived from an acid monomer comprising one or more carboxylic or sulfonic groups, or alternatively B and C can denote groups derived from carboxybetaine or sulfobetaine zwitterionic monomers; B and C can also denote a cationic polymer chain comprising primary, secondary, tertiary or quaternary amine groups, in which at least one of the amine groups bears a carboxylic or sulfonic group connected via a hydrocarbon group or alternatively B and C form part of a chain of a polymer containing an ethylenedicarboxylic unit in which one of the carboxylic groups has been made to react with a polyamine comprising one or more primary or secondary amine groups.

The amphoteric polymers corresponding to the definition given above that are more particularly preferred are chosen from the following polymers:
(1) polymers resulting from the copolymerization of a monomer derived from a vinyl compound bearing a carboxylic group such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylates and acrylates, dialkylaminoalkylmethacrylamides and -acrylamides. Such compounds are described in US patent No. 3 836 537.
   The vinyl compound may also be a dialkyldiallylammonium salt such as diethyldiallylammonium chloride.
(2) polymers comprising units deriving from:
   a) at least one monomer chosen from acrylamides and methacrylamides substituted on the nitrogen with an alkyl group,
   b) at least one acidic comonomer containing one or more reactive carboxylic groups, and
   c) at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

   The N-substituted acrylamides or methacrylamides that are more particularly preferred according to the invention are groups in which the alkyl groups contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
   The acidic comonomers are chosen more particularly from acrylic acid, methacrylic acid, crotonic acid, itaconic acid, maleic acid and fumaric acid and alkyl monoesters, having 1 to 4 carbon atoms, of maleic or fumaric acids or anhydrides. The preferred basic comonomers are aminoethyl, butylaminoethyl, N,N'-dimethylaminoethyl and N-tert-butylaminoethyl methacrylates. The copolymers whose CTFA (4th edition, 1991) name is Octylacrylamide/acrylates/butylaminoethyl methacrylate copolymer, such as the products sold under the name Amphomer® or Lovocryl® 47 by the company National Starch, are particularly used.
(3) crosslinked and alkylated polyamino amides partially or totally derived from polyamino amides of general formula: 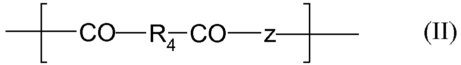 in which R₄ represents a divalent group derived from a saturated dicarboxylic acid, a mono- or dicarboxylic aliphatic acid bearing an ethylenic double bond, an ester of an alcohol containing 1 to 6 carbon atoms, of these acids, or a group derived from the addition of any one of the said acids to a bis(primary) amine or bis(secondary) derivative, and Z denotes a group from a bis(primary), mono- or bis(secondary) polyalkylene polyamine and preferably represents:
   a) in proportions of from 60 to 100 mol%, the group: 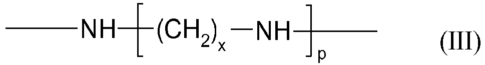 where x = 2 and p = 2 or 3, or alternatively x = 3 and p = 2
      this group being derived from diethylenetriamine, triethylenetetramine or dipropylenetriamine;
   b) in proportions of from 0 to 40 mol%, the group (III) above in which x = 2 and p = 1 and which is derived from ethylenediamine, or the group derived from piperazine: 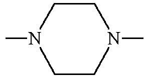
   c) in proportions of from 0 to 20 mol%, the -NH(CH₂)₆-NH-group derived from hexamethylenediamine, these polyaminoamines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof.
      The saturated carboxylic acids are preferably chosen from acids having 6 to 10 carbon atoms, such as adipic acid, 2,2,4-trimethyladipic acid and 2,4,4-trimethyladipic acid, terephthalic acid, acids containing an ethylenic double bond such as, for example, acrylic acid, methacrylic acid and itaconic acid. The alkane sultones used in the alkylation are preferably propane sultone or butane sultone, the salts of the alkylating agents are preferably the sodium or potassium salts.
(4) polymers comprising zwitterionic units of formula: 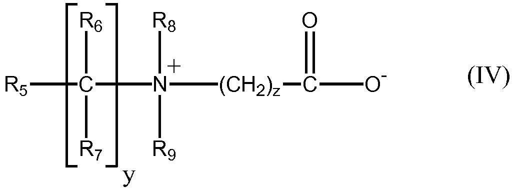 in which R₅ denotes a polymerizable unsaturated group such as an acrylate, methacrylate, acrylamide or methacrylamide group, y and z each represent an integer from 1 to 3, R₆ and R₇ represent a hydrogen atom, a methyl, ethyl or propyl group, R₈ and R₉ represent a hydrogen atom or an alkyl group such that the sum of the carbon atoms in R₁₀ and R₁₁ does not exceed 10.
   The polymers comprising such units can also comprise units derived from non-zwitterionic monomers such as dimethyl- or diethylaminoethyl acrylate or methacrylate or alkyl acrylates or methacrylates, acrylamides or methacrylamides or vinyl acetate.
   Mention may be made, by way of example, of methyl methacrylate/methyl dimethylcarboxymethylammonioethyl methacrylate copolymers, such as the product sold under the name Diaformer Z301 by Sandoz.
(5) polymers derived from chitosan comprising monomer units corresponding to the following formulae: 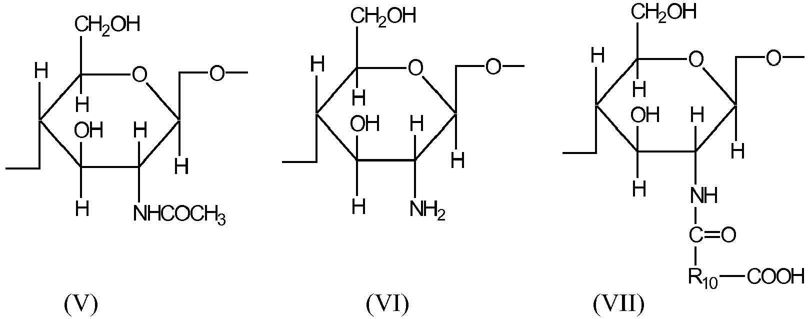 the unit (V) being present in proportions of between 0 and 30%, the unit (VI) in proportions of between 5% and 50% and the unit (VII) in proportions of between 30% and 90%, it being understood that, in this unit (VII), R₁₀ represents a group of formula: 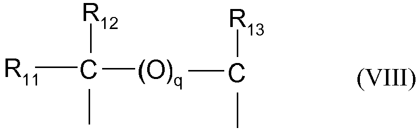 in which, if q = 0, R₁₁, R₁₂ and R₁₃, which are identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted by one or more amine, hydroxyl, carboxyl, alkylthio or sulfonic groups, an alkylthio residue in which the alkyl group bears an amino residue, at least one of the groups R₁₇, R₁₈ and R₁₉ being, in this case, a hydrogen atom;
   or, if q=1, R₁₁, R₁₂ and R₁₃ each represent a hydrogen atom, as well as the salts formed by these compounds with bases or acids.
(6) polymers of units corresponding to the general formula (IX) described, for example, in French patent 1 400 366: 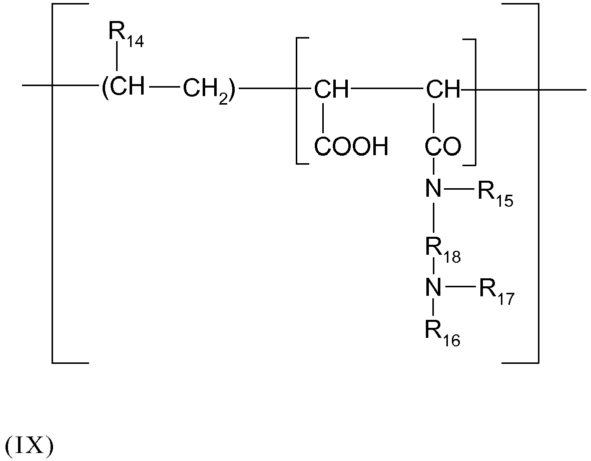 in which R₁₄ represents a hydrogen atom, a CH₃O, CH₃CH₂O or phenyl group, R₁₅ denotes a hydrogen atom or a C₁₋₄ alkyl group such as methyl or ethyl, R₁₆ denotes a hydrogen atom or a C₁₋₄ alkyl group such as methyl or ethyl, R₁₇ denotes a C₁₋₄ alkyl group such as methyl or ethyl or a group corresponding to the formula: -R₁₈-N(R₁₆)₂, R₁₈ representing a -CH₂-CH₂-, -CH₂-CH₂-CH₂- or -CH₂-CH(CH₃)- group and R₁₆ having the abovementioned meanings.
(7) amphoteric polymers of the type -D-X-D-X- chosen from:
   a) polymers obtained by the action of chloroacetic acid or sodium chloroacetate on compounds comprising at least one unit of formula:

      -D-X-D-X-D- (X)

      where D denotes a group 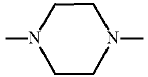 and X denotes the symbol E or E', E or E', which may be identical or different, denote a divalent group that is an alkylene group with a straight or branched chain containing up to 7 carbon atoms in the main chain, which is unsubstituted or substituted with hydroxyl groups and which can comprise, in addition to the oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
   b) polymers of formula:

      -D-X-D-X- (XI)

      where D denotes a group 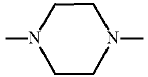 and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and containing one or more nitrogen atoms, the nitrogen atom being substituted with an alkyl chain that is optionally interrupted by an oxygen atom and necessarily comprising one or more carboxyl functions or one or more hydroxyl functions and betainized by reaction with chloroacetic acid or sodium chloroacetate.
(8) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkanolamine. These copolymers can also comprise other vinyl comonomers such as vinylcaprolactam.

According to a preferred embodiment of the invention, the amphoteric fixing polymers that may be used in the composition according to the invention may be chosen from those of family (3), such as the copolymers whose CTFA name is Octylacrylamide/acrylates/butylaminoethyl methacrylate copolymer, such as the products sold under the name Amphomer®, Amphomer® LV 71 or Lovocryl® 47 by the company National Starch and those of family (4) such as the methyl methacrylate/methyl dimethylcarboxymethylammonioethyl methacrylate copolymers, sold, for example, under the name Diaformer Z301 by the company Sandoz.

The nonionic fixing polymers that may be used according to the present invention are chosen, for example, from:
- polyalkyloxazolines;
- vinyl acetate homopolymers;
- vinyl acetate copolymers, for instance copolymers of vinyl acetate and acrylic ester, copolymers of vinyl acetate and ethylene, or copolymers of vinyl acetate and maleic ester, for example dibutyl maleate,
- homopolymers and copolymers of acrylic esters, for instance copolymers of alkyl acrylates and alkyl methacrylates, such as the products provided by Rohm & Haas under the names Primal® AC-261 K and Eudragit® NE 30 D, by BASF under the name 8845 or by Hoechst under the name Appretan® N9212,
- copolymers of acrylonitrile and a nonionic monomer chosen, for example, from butadiene and alkyl (meth)acrylates, such as the products provided under the name CJ 0601 B by Rohm & Haas,
- styrene homopolymers;
- styrene copolymers, for instance copolymers of styrene and alkyl (meth)acrylate, such as the products Mowilith® LDM 6911, Mowilith® DM 611 and Mowilith® LDM 6070, provided by Hoechst, and the products Rhodopas® SD 215 and Rhodopas® DS 910, provided by Rhône-Poulenc, copolymers of styrene, alkyl methacrylate and alkyl acrylate, copolymers of styrene and butadiene, or copolymers of styrene, butadiene and vinylpyridine,
- polyamides,
- vinyllactam homopolymers such as vinylpyrrolidone homopolymers and the polyvinylcaprolactam sold under the name Luviskol® Plus by the company BASF; and
- vinyllactam copolymers such as a poly(vinylpyrrolidone/vinyllactam) copolymer sold under the trade name Luvitec® VPC 55K65W by the company BASF, poly(vinylpyrrolidone/vinyl acetate) copolymers, such as those sold under the name PVPVA® S630L by the company ISP, Luviskol® VA 73, VA 64, VA 55, VA 37 and VA 28 by the company BASF, and poly(vinylpyrrolidone/vinyl acetate/vinyl propionate) terpolymers, for instance the product sold under the name Luviskol® VAP 343 by the company BASF.

The alkyl groups of the abovementioned nonionic polymers preferably have from 1 to 6 carbon atoms.

Preferably, the fixing polymer(s) used in the composition according to the invention are anionic, cationic or nonionic fixing polymers, in particular acrylic acid copolymers such as the acrylic acid/ethyl acrylate/N-tert-butylacrylamide terpolymers sold especially under the name Ultrahold® Strong by the company BASF; quaternary copolymers of vinylpyrrolidone and of vinylimidazole, such as the vinylimidazolium methosulfate/vinylcaprolactam/vinylpyrrolidone terpolymer sold under the name Luviquat Hold by the company BASF; poly(vinylamine/N-vinylformamide) copolymers such as the product sold under the name Luviquat 9030 by the company BASF; and vinyllactam copolymers, such as the poly(vinylpyrrolidone/vinyl acetate) copolymers sold under the name Luviskol® VA 64 by the company BASF.

The fixing polymer(s) are present in an amount of greater than 0.1% by weight, preferably ranging from 0.1% to 20% by weight, better still from 0.5% to 15% by weight and even more preferentially from 0.5% to 8% by weight relative to the total weight of the composition comprising phases (i) and (ii).

The fatty phase (ii) of the composition according to the invention is insoluble in phase (i), i.e. insoluble in the alcoholic or aqueous-alcoholic medium at room temperature (25°C) and at atmospheric pressure (760 mmHg, i.e. 1.013 × 10⁵ Pa), i.e. with a weight solubility of less than 5%, preferably less than 1% and even more preferentially less than 0.1%.

The fatty phase (ii) of the composition according to the invention comprises one or more fatty substances.

Preferably, the fatty phase (ii) consists of one or more fatty substances and possibly of one or more compounds soluble in at least one of the fatty substances of the fatty phase, at room temperature (25°C) and at atmospheric pressure (760 mmHg, i.e. 1.013 × 10⁵ Pa). The term "soluble" means a weight solubility greater than 0.5%, preferably greater than 1%, even more preferably greater than 5%.

According to one specific embodiment, the fatty phase comprises exclusively one or more fatty substances.

The term "fatty substance" means an organic compound that is insoluble in water at room temperature (25°C) and at atmospheric pressure (760 mmHg, i.e. 1.013 × 10⁵ Pa), i.e. with a weight solubility of less than 5%, preferably less than 1% and even more preferentially less than 0.1%.

The composition according to the invention preferably comprises at least one fatty substance that is liquid at room temperature and at atmospheric pressure.

The liquid fatty substances of the invention preferably have a viscosity of less than or equal to 2 Pa.s, better still less than or equal to 1 Pa.s and even better still less than or equal to 0.1 Pa.s at a temperature of 25°C and at a shear rate of 1 s⁻¹.

The fatty substances of the invention that are liquid at room temperature and at atmospheric pressure are more preferentially chosen from hydrocarbons, fatty alcohols, fatty esters, fatty ethers and silicone oils, and mixtures thereof.

Even more preferentially, they are chosen from hydrocarbons, fatty esters and silicone oils, and mixtures thereof.

The fatty substances of the invention are different from (poly)oxyalkylenated and (poly)glycerolated ethers and also from salified fatty acids.

The term "liquid hydrocarbon" means a hydrocarbon composed solely of carbon and hydrogen atoms, which is liquid at room temperature (25°C) and at atmospheric pressure (760 mmHg, i.e. 1.013 × 10⁵ Pa), which is especially of mineral or plant origin, preferably of plant origin.

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane,
- linear or branched hydrocarbons of mineral, animal or synthetic origin, of more than 16 carbon atoms, such as volatile or non-volatile liquid paraffins, liquid petroleum jelly, polydecenes, hydrogenated polyisobutene such as the product sold under the brand name Parleam® by the company NOF Corporation, and squalane.

In one preferred variant, the liquid hydrocarbon(s) are chosen from volatile or non-volatile liquid paraffins, and derivatives thereof, and liquid petroleum jelly.

The term "liquid fatty alcohol" means a non-glycerolated and non-oxyalkylenated fatty alcohol, which is liquid at room temperature (25°C) and at atmospheric pressure (760 mmHg, i.e. 1.013 × 10⁵ Pa).

Preferably, the liquid fatty alcohols of the invention comprise from 8 to 30 carbon atoms.

The liquid fatty alcohols of the invention can be saturated or unsaturated.

The saturated liquid fatty alcohols are preferably branched. They can optionally comprise, in their structure, at least one aromatic or non-aromatic ring. They are preferably acyclic.

More particularly, the saturated liquid fatty alcohols of the invention are chosen from octyldodecanol, isostearyl alcohol and 2-hexyldecanol.

Octyldodecanol is very particularly preferred.

The unsaturated liquid fatty alcohols exhibit, in their structure, at least one double or triple bond and preferably one or more double bonds. When several double bonds are present, there are preferably 2 or 3 of them and they can be conjugated or unconjugated.

These unsaturated fatty alcohols can be linear or branched.

They can optionally comprise, in their structure, at least one aromatic or non-aromatic ring. They are preferably acyclic.

More particularly, the unsaturated liquid fatty alcohols of the invention are chosen from oleyl alcohol, linoleyl alcohol, linolenyl alcohol and undecylenyl alcohol.

Oleyl alcohol is very particularly preferred.

The term "liquid fatty ester" means an ester derived from a fatty acid and/or from a fatty alcohol that is liquid at room temperature (25°C) and at atmospheric pressure (760 mmHg, i.e. 1.013 × 10⁵ Pa).

The esters are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

The composition may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars can be monosaccharides, oligosaccharides or polysaccharides.

Examples of suitable sugars that may be mentioned include saccharose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and in particular of sucrose, glucose or methylglucose mono- or dioleates, stearates, behenates, oleopalmitates, linoleates, linolenates or oleostearates.

An example that may be mentioned is the product sold under the name Glucate® DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, olive oil, rapeseed oil, coconut oil, wheatgerm oil, sweet almond oil, safflower oil, candlenut oil, camellina oil, tamanu oil, babassu oil and pracaxi oil, caprylic/capric acid triglycerides, for instance those sold by the company Stéarineries Dubois or those sold under the names Miglyol® 810, 812 and 818 by the company Dynamit Nobel, jojoba oil and shea butter oil.

Liquid fatty esters derived from monoalcohols will preferably be used as esters according to the invention.

Isopropyl myristate and isopropyl palmitate are particularly preferred.

The liquid fatty ethers are chosen from liquid dialkyl ethers such as dicaprylyl ether.

The term "silicone oil" means an oil containing at least one silicon atom, and especially containing Si-O groups. The silicone oil may be chosen from non-volatile silicone oils and volatile silicone oils, and mixtures thereof.

Volatile silicone oil that may be used in the invention may be chosen from silicone oils with a flash point ranging from 40°C to 102°C, preferably with a flash point of greater than 55°C and less than or equal to 95°C and preferentially ranging from 65°C to 95°C. Volatile silicone oils that may be mentioned include linear or cyclic silicone oils containing from 2 to 7 silicon atoms, these silicones optionally comprising alkyl or alkoxy groups containing from 1 to 10 carbon atoms. Examples of volatile silicone oils that may especially be mentioned include cyclopolydimethylsiloxanes (INCI name: cyclomethicone), such as cyclopentasiloxane, cyclohexasiloxane, octamethylcyclotetrasiloxane, decamethylcyclopentasiloxane, dodecamethylcyclohexasiloxane; linear silicones such as heptamethylhexyltrisiloxane, heptamethyloctyltrisiloxane, hexamethyldisiloxane, octamethyltrisiloxane, decamethyltetrasiloxane and dodecamethylpentasiloxane; and mixtures thereof.

In one embodiment, the non-volatile silicone oil that may be used in the invention may be chosen from polymethylsiloxanes (PDMS) and phenyl polymethylsiloxanes such as phenyl trimethicones, phenyl dimethicones, phenyltrimethylsiloxydiphenylsiloxanes, diphenyl dimethicones, diphenylmethyldiphenyltrisiloxanes and polymethylphenylsiloxanes; polysiloxanes modified with fatty acids or fatty alcohols, and mixtures thereof.

Preferably, the fatty substances are chosen from hydrocarbons such as isododecane and isohexadecane, liquid petroleum jelly, fatty esters derived from monoalcohols, such as C₂₋₄ alkyl myristates, in particular isopropyl myristate, silicone oils such as cyclopentadimethylsiloxane and phenyl trimethicone, and mixtures thereof.

Preferably, the fatty substance(s) used in the invention are insoluble in phase (i), i.e. insoluble in the alcoholic or aqueous-alcoholic medium at room temperature (25°C) and at atmospheric pressure (760 mmHg, i.e. 1.013 × 10⁵ Pa), i.e. with a weight solubility of less than 5%, preferably less than 1% and even more preferentially less than 0.1%.

In particular, in one embodiment, when the medium of phase (i) is aqueous-alcoholic, the fatty substances as described above are all in the fatty phase (ii).

In another embodiment, certain fatty substances may be at least partially soluble in the alcoholic or aqueous-alcoholic medium and may thus be at least partially present in phase (i).

In particular, when the medium of phase (i) is alcoholic, certain fatty substances may be soluble in the alcoholic medium and may thus be at least partially present in phase (i).

The fatty substance(s) are present in an amount preferably ranging from 5% to 90% by weight, better still from 5% to 55% by weight and even more preferentially from 10% to 30% by weight relative to the total weight of the composition comprising phases (i) and (ii).

The composition of the invention may also comprise solid fatty substances that are soluble in the liquid fatty substances, such as solid fatty alcohols, solid fatty esters, solid fatty amides and in particular ceramides.

The cosmetic compositions defined in the invention may also contain one or more additives chosen from glycerol, fragrances, dyes and alkaline agents.

These additives may be present in the composition according to the invention in an amount ranging from 0 to 20% by weight relative to the total weight of the composition.

A person skilled in the art will take care to select these optional additives and the amounts thereof such that they do not harm the properties of the compositions of the present invention.

The compositions in accordance with the invention may be conditioned in a pump bottle that is common in cosmetics. Another subject-matter of the invention is therefore a pump bottle containing the cosmetic composition according to the invention.

The pump bottle comprises a container equipped with a pump and on which is mounted a push-button configured to actuate the pump, the push-button also being equipped with a nozzle, preferably with channels, making it possible to supply the compositions in the form of droplets. The sprayed compositions are in the form of a spray.

The present invention also relates to a process for shaping and/or holding the hairstyle, comprising the use of a cosmetic composition as described above.

In particular, the process for shaping and/or holding the hairstyle comprises a step of applying to the hair a cosmetic composition as defined previously, sprayed from a pump bottle.

The present invention also relates to the use of the cosmetic composition according to the invention, sprayed from the pump bottle, for shaping and/or holding the hairstyle.

The examples that follow serve to illustrate the invention.

### EXAMPLES

In the examples that follow, all the amounts are shown as weight percentages of product relative to the total weight of the composition.

### Example 1

The composition below according to the invention was prepared from the compounds indicated in the table below.

| | Amount in weight% |
|---|---|
| Aqueous-alcoholic phase (i) | |
| Acrylic acid/ethyl acrylate/n-tert-butylacrylamide terpolymer¹ | 5 |
| 2-Amino-2-methyl-1-propanol | 0.6 |
| Water | 15 |
| Denatured absolute ethanol | 59.4 |
| Fatty phase (ii) | |
| Isopropyl myristate | 20 |

| | |
|---|---|
| ¹: Sold under the trade name Ultrahold Strong by BASF | |

The constituents of the aqueous-alcoholic phase are mixed together, and the fatty phase is then mixed with the aqueous-alcoholic phase.

A composition is obtained, which, when at rest, comprises an aqueous-alcoholic phase and a separate fatty phase, the fatty phase being above the aqueous-alcoholic phase.

### Example 2

The composition below according to the invention was prepared from the compounds indicated in the table below.

| | Amount in weight% |
|---|---|
| Aqueous-alcoholic phase (i) | |
| Vinylimidazolium methosulfate/vinylcaprolactam/vinylpyrrolidone terpolymer² | 0.8 |
| Vinylpyrrolidone/vinyl acetate copolymer (60/40)³ | 2 |
| Water | 23.2 |
| Denatured absolute ethanol | 54 |
| Fatty phase (ii) | |
| Isopropyl myristate | 20 |

| | |
|---|---|
| ²: Sold under the trade name Luviquat Hold by BASF ³: Sold under the trade name Luviskol VA64 W by BASF | |

The constituents of the aqueous-alcoholic phase are mixed together, and the fatty phase is then mixed with the aqueous-alcoholic phase.

A composition is obtained, which, when at rest, comprises an aqueous-alcoholic phase and a separate fatty phase, the fatty phase being above the aqueous-alcoholic phase.

### Comparative Example 1

The following one-phase composition was prepared from the compounds indicated in the table below.

| | Amount in weight% |
|---|---|
| Acrylic acid/ethyl acrylate/n-tert-butylacrylamide terpolymer ⁶ | 5 |
| 2-Amino-2-methyl-1-propanol | 0.6 |
| Cyclopentadimethylsiloxane | 23 |
| Phenyl trimethicone | 18 |
| Denatured absolute ethanol | 53.4 |

| | |
|---|---|
| ⁶: Sold under the trade name Ultrahold Strong by BASF | |

The compounds indicated in the above table are mixed together and a one-phase composition is obtained.

This composition, and also those of Examples 1 and 2, were then placed in identical pump bottles. The composition of Example 1 was sprayed onto dried hair.

The same procedure was followed with the compositions of Example 2 and of Comparative Example 1 on other locks.

A panel of experts then noted the fixing of the hairstyle by attributing grades ranging from 0 to 5, with 0 corresponding to insufficient fixing of the hairstyle and 5 corresponding to high fixing of the hairstyle. The panel of testers also evaluated the sheen of the hair by attributing grades ranging from 0 to 5.

The results are collated in the table below.

| | Sheen | Fixing |
|---|---|---|
| Spray of Example 1 (invention) | 3.0 | 1.6 |
| Spray of Example 2 (invention) | 2.9 | 1.8 |
| Spray of Comparative Example 1 | 2.9 | 0.9 |

It is noted that the cosmetic compositions according to the invention of Examples 1 and 2 give sheen equivalent to that imparted by the comparative composition (Comparative Example 1) but with a markedly better level of fixing.

### Example 3

The composition below according to the invention was prepared from the compounds indicated in the table below.

| | Amount in weight% |
|---|---|
| Aqueous-alcoholic phase (i) | |
| Polyvinylamine/N-vinylformamide copolymer⁴ | 1 |
| Water | 25 |
| Denatured absolute ethanol | 54 |
| Fatty phase (ii) | |
| Isopropyl myristate | 20 |

| | |
|---|---|
| ⁴: Sold under the trade name Luviquat 9030 by BASF | |

The constituents of the aqueous-alcoholic phase are mixed together, and the fatty phase is then mixed with the aqueous-alcoholic phase.

A composition is obtained, which, when at rest, comprises an aqueous-alcoholic phase and a separate fatty phase, the fatty phase being above the aqueous-alcoholic phase. After spraying the composition from a pump bottle onto dried hair, it is found that the hair is shiny while at the same time having good fixing of the hairstyle.

### Example 4

The composition below according to the invention was prepared from the compounds indicated in the table below.

| | Amount in weight% |
|---|---|
| Alcoholic phase (i) | |
| Acrylic acid/ethyl acrylate/n-tert-butylacrylamide terpolymer ⁵ | 4 |
| 2-Amino-2-methyl-1-propanol | 0.5 |
| Denatured absolute ethanol | 54.5 |
| Cyclopentadimethylsiloxane | 19 |
| Fatty phase (ii) | |
| Liquid petroleum jelly | 22 |

| | |
|---|---|
| ⁵: Sold under the trade name Ultrahold Strong by BASF | |

The constituents of the alcoholic phase are mixed together, and the fatty phase is then mixed with the alcoholic phase.

An anhydrous composition is obtained, which, when at rest, comprises an alcoholic phase and a separate fatty phase, the alcoholic phase being above the fatty phase.

After spraying the composition from a pump bottle onto dried hair, it is found that the hair is shiny while at the same time having good fixing of the hairstyle.

## Claims

1. Cosmetic composition comprising two or more than two separate liquid phases:
(i) one of the liquid phases comprising an alcoholic or aqueous-alcoholic medium and one or more fixing polymers that are soluble in the said medium, and
(ii) the other of the liquid phases being a fatty phase that is insoluble in phase (i) and comprising one or more fatty substances,
the phase (i) comprising from 0 to 50% by weight of water relative to the total weight of phase (i).

2. Cosmetic composition according to Claim 1, **characterized in that** the fatty phase (ii) is above the alcoholic or aqueous-alcoholic phase (i).

3. Cosmetic composition according to either of the preceding claims, **characterized in that** the weight ratio of phase (i) to phase (ii) ranges from 0.1 to 20 and better still from 0.8 to 20.

4. Cosmetic composition according to any one of the preceding claims, **characterized in that** the alcoholic or aqueous-alcoholic medium comprises one or more C₁-C₆ alcohols, such as ethanol.

5. Cosmetic composition according to any one of the preceding claims, **characterized in that** phase (i) comprises from 10% to 50% by weight of water relative to the total weight of phase (i).

6. Cosmetic composition according to any one of the preceding claims, **characterized in that** the fixing polymer(s) are chosen from anionic, cationic and nonionic fixing polymers, in particular from acrylic acid copolymers such as acrylic acid/ethyl acrylate/N-tert-butylacrylamide terpolymers; quaternary copolymers of vinylpyrrolidone and of vinylimidazole, such as the vinylimidazolium methosulfate/vinylcaprolactam/vinylpyrrolidone terpolymer; poly(vinylamine/N-vinylformamide) copolymers, and vinyllactam copolymers, such as poly(vinylpyrrolidone/vinyl acetate) copolymers.

7. Cosmetic composition according to any one of the preceding claims, **characterized in that** the fixing polymer(s) are present in an amount of greater than 0.1% by weight, preferably ranging from 0.1% to 20% by weight relative to the total weight of the composition.

8. Cosmetic composition according to any one of the preceding claims, **characterized in that** it comprises at least one liquid fatty substance chosen from hydrocarbons, fatty esters and silicone oils, and mixtures thereof.

9. Cosmetic composition according to any one of the preceding claims, **characterized in that** it comprises at least one liquid fatty substance chosen from isododecane, isohexadecane, liquid petroleum jelly, fatty esters derived from monoalcohols, such as C₂₋₄ alkyl myristates, in particular isopropyl myristate, and phenyl trimethicone, and mixtures thereof.

10. Cosmetic composition according to any one of the preceding claims, **characterized in that** the fatty substance(s) are present in an amount ranging from 5% to 90% by weight, better still from 5% to 55% by weight and even more preferentially from 10% to 30% by weight relative to the total weight of the composition.

11. Cosmetic composition according to any one of the preceding claims, **characterized in that** the fatty substance(s) are insoluble in phase (i).

12. Cosmetic composition according to any one of the preceding claims, **characterized in that** it comprises one or more additives chosen from glycerol, fragrances, dyes and alkaline agents.

13. Pump bottle containing a cosmetic composition according to any one of the preceding claims.

14. Cosmetic process for treating keratin materials, comprising a step of applying to the hair a cosmetic composition as defined in any one of Claims 1 to 12, sprayed from a pump bottle.

15. Use of the cosmetic composition according to any one of Claims 1 to 12, sprayed from a pump bottle, for treating keratin materials.

## Patentansprüche

1. Kosmetische Zusammensetzung, umfassend zwei oder mehr als zwei separate flüssige Phasen:
(i) wobei eine der flüssigen Phasen ein alkoholisches oder wässrig-alkoholisches Medium und ein oder mehrere fixierende Polymere, die in dem Medium löslich sind, umfasst, und
(ii) wobei es sich bei der anderen der flüssigen Phasen um eine Fettphase handelt, die in Phase (i) unlöslich ist und eine oder mehrere Fettsubstanzen umfasst,
wobei die Phase (i) 0 bis 50 Gew.-% Wasser, bezogen auf das Gesamtgewicht von Phase (i), umfasst.

2. Kosmetische Zusammensetzung nach Anspruch 1, **dadurch gekennzeichnet, dass** sich die Fettphase (ii) über der alkoholischen oder wässrigalkoholischen Phase (i) befindet.

3. Kosmetische Zusammensetzung nach einem der vorhergehenden Ansprüche, **dadurch gekennzeichnet, dass** das Gewichtsverhältnis von Phase (i) zu Phase (ii) im Bereich von 0,1 bis 20 und noch besser von 0,8 bis 20 liegt.

4. Kosmetische Zusammensetzung nach einem der vorhergehenden Ansprüche, **dadurch gekennzeichnet, dass** das alkoholische oder wässrig-alkoholische Medium einen oder mehrere C₁-C₆-Alkohole, wie Ethanol, umfasst.

5. Kosmetische Zusammensetzung nach einem der vorhergehenden Ansprüche, **dadurch gekennzeichnet, dass** Phase (i) 10 bis 50 Gew.-% Wasser, bezogen auf das Gesamtgewicht von Phase (i), umfasst.

6. Kosmetische Zusammensetzung nach einem der vorhergehenden Ansprüche, **dadurch gekennzeichnet, dass** das fixierende Polymer bzw. die fixierenden Polymere aus anionischen, kationischen und nichtionischen fixierenden Polymeren, insbesondere aus Acrylsäure-Copolymeren wie Acrylsäure/Ethylacrylat/N-tert-Butylacrylamid-Terpolymeren; quaternären Copolymeren von Vinylpyrrolidon und von Vinylimidazol, wie dem Vinylimidazoliummethosulfat/Vinylcaprolactam/Vinylpyrrolidon-Terpolymer; Poly(vinylamin/N-Vinylformamid)-Copolymeren und Vinyllactam-Copolymeren, wie Poly(vinylpyrrolidon/vinylacetat)-Copolymeren, ausgewählt ist bzw. sind.

7. Kosmetische Zusammensetzung nach einem der vorhergehenden Ansprüche, **dadurch gekennzeichnet, dass** das fixierende Polymer bzw. die fixierenden Polymere in einer Menge von mehr als 0,1 Gew.-%, vorzugsweise im Bereich von 0,1 bis 20 Gew.-%, bezogen auf das Gesamtgewicht der Zusammensetzung, vorliegt bzw. vorliegen.

8. Kosmetische Zusammensetzung nach einem der vorhergehenden Ansprüche, **dadurch gekennzeichnet, dass** sie mindestens eine flüssige Fettsubstanz, die aus Kohlenwasserstoffen, Fettestern und Silikonölen und Mischungen davon ausgewählt ist, umfasst.

9. Kosmetische Zusammensetzung nach einem der vorhergehenden Ansprüche, **dadurch gekennzeichnet, dass** sie mindestens eine flüssige Fettsubstanz, die aus Isododecan, Isohexadecan, Vaselineölen, von Monoalkoholen abgeleiteten Fetttestern, wie C₂₋₄-Alkyl-myristaten, insbesondere Isopropylmyristat, und Phenyltrimethicon und Mischungen davon ausgewählt ist, umfasst.

10. Kosmetische Zusammensetzung nach einem der vorhergehenden Ansprüche, **dadurch gekennzeichnet, dass** die Fettsubstanz bzw. die Fettsubstanzen in einer Menge im Bereich von 5 bis 90 Gew.-%, noch besser von 5 bis 55 Gew.-% und noch weiter bevorzugt von 10 bis 30 Gew.-%, bezogen auf das Gesamtgewicht der Zusammensetzung, vorliegt bzw. vorliegen.

11. Kosmetische Zusammensetzung nach einem der vorhergehenden Ansprüche, **dadurch gekennzeichnet, dass** die Fettsubstanz bzw. die Fettsubstanzen in Phase (i) unlöslich ist bzw. sind.

12. Kosmetische Zusammensetzung nach einem der vorhergehenden Ansprüche, **dadurch gekennzeichnet, dass** sie ein oder mehrere Additive, die aus Glycerin, Duftstoffen, Farbstoffen und alkalischen Mitteln ausgewählt sind, umfasst.

13. Pumpflasche, die eine kosmetische Zusammensetzung nach einem der vorhergehenden Ansprüche enthält.

14. Kosmetisches Verfahren zur Behandlung von Keratinmaterialien, bei dem man auf das Haar eine aus einer Pumpflasche versprühte kosmetische Zusammensetzung gemäß einem der Ansprüche 1 bis 12 aufbringt.

15. Verwendung der kosmetischen Zusammensetzung nach einem der Ansprüche 1 bis 12, die aus einer Pumpflasche versprüht wird, zur Behandlung von Keratinmaterialien.

## Revendications

1. Composition cosmétique comprenant deux ou plus de deux phases liquides distinctes :
(i) l'une des phases liquides comprenant un milieu alcoolique ou hydroalcoolique et un ou plusieurs polymères fixants solubles dans ledit milieu, et
(ii) l'autre des phases liquides étant une phase grasse insoluble dans la phase (i) et comprenant un ou plusieurs corps gras,
la phase (i) comprenant de 0 à 50 % en poids d'eau par rapport au poids total de la phase (i).

2. Composition cosmétique selon la revendication 1, **caractérisée en ce que** la phase grasse (ii) est au-dessus de la phase alcoolique ou hydro-alcoolique (i).

3. Composition cosmétique selon l'une quelconque des revendications précédentes, **caractérisée en ce que** le rapport pondéral de la phase (i) sur la phase (ii) varie de 0,1 à 20, et mieux de 0,8 à 20.

4. Composition cosmétique selon l'une quelconque des revendications précédentes, **caractérisée en ce que** le milieu alcoolique ou hydroalcoolique comprend un ou plusieurs alcools en C₁-C₆ comme l'éthanol.

5. Composition cosmétique selon l'une quelconque des revendications précédentes, **caractérisée en ce que** la phase (i) comprend de 10 à 50 % en poids d'eau par rapport au poids total de la phase (i).

6. Composition cosmétique selon l'une quelconque des revendications précédentes, **caractérisée en ce que** le(s) polymère(s) fixant(s) est ou sont choisi(s) parmi des polymères fixants anioniques, cationiques ou non ioniques, en particulier parmi les copolymères d'acide acrylique tels que les terpolymères acide acrylique/acrylate d'éthyle/N-tertiobutylacrylamide ; les copolymères quaternaires de vinylpyrrolidone et de vinylimidazole comme le terpolymère méthosulfate de vinylimidazolium/ vinylcaprolactame/ vinylpyrrolidone ; les copolymères polyvinylamine/N-vinylformamide et les copolymères de vinyllactame, tels que les copolymères poly(vinylpyrrolidone/acétate de vinyle).

7. Composition cosmétique selon l'une quelconque des revendications précédentes, **caractérisée en ce que** le(s) polymère(s) fixant(s) est ou sont présent(s) en une quantité supérieure à 0,1 % en poids, de préférence allant de 0,1 à 20 % en poids par rapport au poids total de la composition.

8. Composition cosmétique selon l'une quelconque des revendications précédentes, **caractérisée en ce qu'**elle comprend au moins un corps gras liquide choisi parmi les hydrocarbures, les esters gras, les huiles de silicone et leurs mélanges.

9. Composition cosmétique selon l'une quelconque des revendications précédentes, **caractérisée en ce qu'**elle comprend au moins un corps gras liquide choisi parmi l'isododécane, l'isohexadécane, l'huile de vaseline, les esters gras issus de monoalcools, tels que les myristates d'alkyle en C₂₋₄, en particulier le myristate d'isopropyle, la phényl triméthicone et leurs mélanges.

10. Composition cosmétique selon l'une quelconque des revendications précédentes, **caractérisée en ce que** le(s) corps gras est ou sont présent(s) en une quantité allant de 5 à 90 % en poids, mieux encore de 5 à 55 % en poids, et encore plus préférentiellement de 10 à 30 % en poids par rapport au poids total de la composition.

11. Composition cosmétique selon l'une quelconque des revendications précédentes, **caractérisée en ce que** le(s) corps gras est ou sont insolubles dans la phase (i).

12. Composition cosmétique selon l'une quelconque des revendications précédentes, **caractérisée en ce qu'**elle comprend un ou plusieurs additifs choisis parmi la glycérine, les parfums, les colorants et les agents alcalins.

13. Flacon-pompe contenant une composition cosmétique selon l'une quelconque des revendications précédentes.

14. Procédé de traitement cosmétique des matières kératiniques, comportant une étape d'application sur les cheveux, d'une composition cosmétique telle que définie dans l'une quelconque des revendications 1 à 12, pulvérisée à partir d'un flacon pompe.

15. Utilisation de la composition cosmétique pulvérisée à partir du flacon pompe selon l'une quelconque des revendications 1 à 12, pour le traitement des matières kératiniques.
